# EUROPEAN PATENT SPECIFICATION

(11) **EP 0 639 564 B1**
(45) Date of publication and mention of the grant of the patent: **29.10.1997**
(21) Application number: 94202359.9
(22) Date of filing: 18.08.1994
(51) Int. Cl.: C07C 409/04, C07C 407/00

(54) **Process for the preparaton of tertiary-butyl hydroperoxide containing mixtures**
Verfahren zur Herstellung von tertiär-Butyl-Hydroperoxid enthaltende Mischungen
Procédé pour la préparation de mélanges contenant de l'hydroperoxyde de butyle tertiare

(30) Priority: 19.08.1993 EP 93202448
(43) Date of publication of application: 22.02.1995
(73) Proprietor: SHELL INTERNATIONALE RESEARCH MAATSCHAPPIJ B.V., 2596 HR Den Haag (NL)
(72) Inventor: Hawton, Malcolm John, NL-2596 HR Den Haag (NL)

(56) References cited:
- EP-A- 0 496 624

## Description

The invention relates to a process for the preparation of tertiary-butyl hydroperoxide containing mixtures, which are suitable to be used as feedstock in the epoxidation of olefins.

Tertiary-butyl hydroperoxide (hereinafter "TBHP") is a well known compound which may be used, either as such, or in admixture with one or more other compounds, in various industrial applications, e.g. as catalyst, as initiator in free-radical type reactions, as intermediate in the preparation of oxirane compounds such as propeneoxide (cf. Aldrichimica Acta, 12(4), 63 (1979)).

A conventional method for preparing TBHP, disclosed in US Patent No. 2,845,461, consists in the oxidation of isobutane with the aid of molecular oxygen, at elevated temperature and pressure.

According to US Patent No. 4,408,081, an improved selectivity with respect to the desired TBHP, without sacrifice in isobutane conversion, is obtained by conducting the oxidation process in a cascade of essentially steady-state reaction zones.

In general in these isobutane oxidation processes a mixture of products is formed, containing besides TBHP, unconverted isobutane, tertiary-butanol, water and minor amounts of other by-products, such as acetone, methanol and organic acids.

For some of the known uses for TBHP, it is desirable, or even essential, that the hydroperoxide, or the mixture containing the hydroperoxide, is substantially waterfree. In particular if the (mixture containing) TBHP is to be used for the preparation of oxirane compounds by epoxidation of olefins, it is of paramount importance that the hydroperoxide feedstock does not contain more than insignificant amounts of water, say less than 1000 ppm.

It is known that water can be removed from TBHP by azeotropic distillation with methylene chloride or dichloroethane (cf. Chem. Berichte, 113, 3662-3665 (1980)) or, after addition of toluene, by separation using a Dean-Stark apparatus (cf. J. Org. Chem. 1983, 48, p 3607/8).

It will be appreciated that these methods, especially on a large scale, are economically not very attractive and, as regards the azeotropic drying with dichloroethane, may be hazardous.

It is also known that water can be removed from an aqueous TBHP containing stream by contacting the said stream with a solid absorbant. Again, this procedure, for large scale operations, is economically unattractive, as it requires large amounts of adsorbant material and moreover may be hazardous, depending on the selected adsorbant.

Furthermore it has been proposed to reduce the water content of an aqueous TBHP stream by subjecting the said stream to an extractive distillation with dry tertiary-butanol as extractant. Part of the water in the aqueous tertiary-butanol overhead stream is removed directly, e.g. by decanting, whilst the remaining water is removed by using an entrainer. A disadvantage of this procedure consists in that it requires a considerable number of distillation units and moreover the temperature and pressure conditions prevailing in the extractive distillation unit may cause hazardous decomposition of TBHP.

Surprisingly, it has now been found that by a simple non-hazardous distillation treatment, the water content of mixtures at least containing TBHP, isobutane and water is reduced to such an extent that the resulting TBHP stream is a suitable feedstock for the epoxidation of olefins.

The invention relates to a process for the preparation of TBHP containing mixtures which are suitable to be used as feedstock in the epoxidation of olefins, whereby a starting mixture at least containing TBHP, isobutane and water, is introduced into a distillation zone, the conditions in the said distillation zone being selected such that an overhead stream is formed, containing part of the isobutane and a major part of the water, and a bottom stream containing the remaining part of the isobutane and substantially all of the TBHP.

Owing to the presence of isobutane in the TBHP containing mixtures, the distillation can be carried out such that in forming an isobutane containing overhead stream use is made of the evaporation heat of isobutane to maintain a relatively low temperature in the bottom part of the distillation zone.

Thermal hazards, that would occur if TBHP is processed at higher distillation temperatures, are thus avoided.

Suitable starting materials to be used in the process of the invention include mixtures containing up to 50% by weight of TBHP. Preferred are starting mixtures whereby the amount of TBHP is in the range of 5 to 30% by weight.

The amount of isobutane in the respective starting mixtures considered suitable for the process of the invention may vary considerably, but is usually well above 40% by weight. Preferably, starting mixtures are used in which the amount of isobutane is in the range of 50 to 95% by weight.

In particular in those instances whereby it is envisaged to use the TBHP streams obtained in the process of the invention as feedstock for epoxidation reactions, it is considered advantageous to apply starting mixtures already containing relatively low amounts of water. Hence, during the process of the invention only relatively small amounts of water have to be removed. Suitable mixtures include mixtures containing not more than 5% by weight of water, preferably from 0.3 to 3% by weight.

The available starting mixtures containing TBHP, isobutane and water, often contain in addition one or more further compounds, depending on the origin of the starting mixture.

In practice, they usually contain tertiary-butanol as an additional compound. Typical amounts of tertiary-butanol are in the range of 5 to 15% by weight, calculated on the total starting mixture.

Very good results have been obtained with starting mixtures, originating from a process for the oxidation of isobutane, for example the product stream obtained in the process, disclosed in US Patent No. 4,408,081. This product stream may be used as such, or may be subjected to one or more washing and/or other purification treatments, before being introduced in the distillation zone of the process of the invention.

If desired, the isobutane content of the starting mixture may be adapted, in order to allow a maximal use of the evaporation heat of the compound during the distillation treatment.

A preferred starting mixture consists of the product stream of a process for the oxidation of isobutane, optionally after being washed, e.g. with water or an aqueous alkaline liquid, which stream subsequently has been enriched with sufficient isobutane, to raise the isobutane content of the starting mixture to at least 80% by weight.

According to the invention it is essential that the conditions prevailing in the distillation zone are selected such that part of the isobutane, initially present in the starting mixture is retained in the bottom stream of the distillation zone. Suitable amounts of isobutane retained in the bottom stream include amounts in the range of 3 to 50% by weight, calculated on the amount of isobutane in the starting mixture. Calculated on the said bottom stream the isobutane content is usually in the range of 10 to 75%.

It has been found that, especially with the starting mixtures containing from 0.3 to 3% by weight of water, the conditions selected in the distillation zone, as regards the retained amount of isobutane, allow the formation of an overhead stream containing at least 70% and often more than 85% of the water present in the starting mixture.

Accordingly, the process of the invention is eminently suitable to produce a TBHP containing stream having a very low water content, allowing the stream to be used directly as feedstock in a process for the epoxidation of olefins, in particular of propene.

Moreover the conditions selected for the distillation zone, generally result in the formation of a bottom stream containing at least 95% and often virtually 100% by weight of the TBHP, present in the starting mixture. Recovery of TBHP from the overhead stream, possibly involving hazardous conditions, as regards inflammability is thus minimized, or can even be entirely omitted.

In practice, depending on the exact composition of the starting material, the conditions prevailing in the distillation zone are preferably selected such that the temperature at the bottom end of the said zone is in the range of 45 to 100 °C, most preferably in the range of 55 to 75 °C and that the pressure in the distillation zone is in the range of 1 to 10 bar gauge, most preferably in the range of 3 to 7 bar gauge.

The process of the invention is preferably carried out in a distillation column having from 10 to 25 theoretical trays. It is considered that the calculation of the optimal number of theoretical trays is within the reach of those skilled in the art.

According to a preferred embodiment of the process of the invention, part of the water, preferably at least 50% of the water, present in the overhead stream from the distillation zone, is removed therefrom, e.g. by decantation after partial or complete condensation of the overhead stream.

Preferably, the isobutane containing overhead stream is at least partly recycled to the distillation zone, or to a process for the oxidation of isobutane.

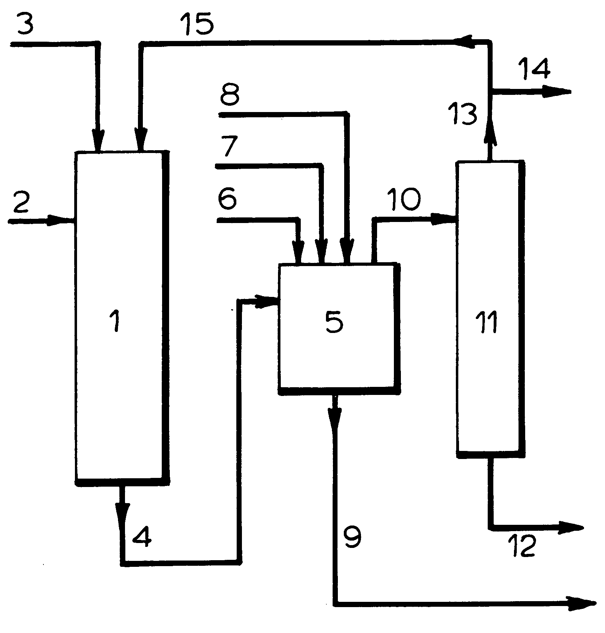

Reference is made to the flow scheme of the attached Figure, depicting a preferred embodiment of the invention. For simplicity, conventional equipment such as boilers, heat exchangers and the likes have not been indicated.

To reactor vessel 1 a continuous stream of isobutane is provided via line 2 and a continuous stream of molecular oxygen via line 3. From the vessel a continuous bottom stream mainly consisting of TBHP, unconverted isobutane, water, and optionally tertiary-butanol, is withdrawn via line 4. The said bottom stream optionally after removal and purification of part of the isobutane contained therein, is introduced into a washing unit 5, where it is washed, e.g. with an aqueous solution of sodium carbonate, supplied via line 6 and with water, supplied via line 7.

Additional isobutane, e.g. (purified) isobutane recovered from the bottom stream of vessel 1 or from a separate source, is supplied via line 8. An effluent stream, mainly consisting of water and entrained contaminants, is withdrawn from the washing unit via line 9.

A washed, isobutane-enriched stream, additionally containing TBHP water, and optionally, tertiary-butanol is withdrawn from the aforesaid washing unit via line 10 and introduced into distillation column 11.

From the distillation column via line 12 a product steam is withdrawn containing TBHP, isobutane and, if any, tertiary-butanol and minor amounts of water and organic contaminants. Via line 13 an overhead stream is withdrawn containing isobutane, most of the water, and if any minor amounts of tertiary-butanol and organic contaminants. From this stream an aqueous stream is separated, e.g. in a decanter (not shown) and removed via line 14. The remaining part of the overhead stream is recycled to the reactor vessel 1 via line 15.

The invention is further illustrated by the following Example.

### Example

An hourly stream of 419 ton, originating from a process for the oxidation of isobutane and consisting of 86.9%w of isobutane, 8.2%w of TBHP, 3.7%w of tertiary-butanol, 0.5%w of water, 0.5%w of acetone and 0.2%w of methanol was supplied to a wash train. Also supplied to the wash train were 1 ton per hour of an aqueous solution of sodium carbonate (23%) and 9 ton per hour of water.

The wash train was operated at a temperature of 50 °C and a pressure of 10 barg. An aqueous effluent steam of 12 ton per hour consisting of 96.1%w of water, 0.4%w of isobutane, 0.6%w of TBHP, 0.9%w of tertiary-butanol, 0.5%w of acetone and 1.5%w of methanol.

The washed hourly stream of 417 ton consisted of 87.2 %w of isobutane, 8 %w of TBHP, 3.7 %w of tertiary-butanol, 0.5 %w of acetone, 0.1 %w of methanol and 0.3 %w of water.

This stream was introduced above tray 9 of a 15 tray distillation column. The bottom temperature was 72 °C, the bottom pressure 4.6 barg.

At an overhead temperature of 40 °C and a pressure of 4.4 barg a vapour stream of 345 ton per hour was obtained.

After condensation from this stream hourly 1 ton was removed, consisting of 96.6%w of water, 0.3%w of isobutane, 0.5%w of acetone 2.6%w of methanol, and only traces of teriary-butanol.

The remaining part of the overhead stream consisted of 99.5 %w of isobutane, 0.1 %w of tertiary-butanol, 0.3 %w of acetone, 0.1 %w of methanol and only traces of water.

The bottom steam withdrawn from the distillation column, 70 ton per hour, consisted of 28.4 %w of isobutane, 48.5 %w of TBHP, 21.7 %w of tertiary-butanol, 1.2 %w of acetone, 0.2 %w of methanol and 88 parts per million of water.

The bottom stream, as regards TBHP and water content, fulfils the requirements to be used as feedstock in a process for converting propene into propene oxide.

## Claims

1. Process for the preparation of tertiary-butyl hydroperoxide containing mixtures which are suitable to be used as feedstock in the epoxidation of olefins, whereby a starting mixture at least containing tertiary-butyl hydroperoxide, isobutane and water, is introduced into a distillation zone, the conditions in the said distillation zone being selected such that an overhead stream is formed, containing part of the isobutane and a major part of the water, and a bottom stream containing the remaining part of the isobutane and substantially all of the tertiary-butyl hydroperoxide.

2. Process as claimed in claim 1, wherein a starting mixture is used in which the amount of tertiary-butyl hydroperoxide is in the range of 5 to 30% by weight.

3. Process as claimed in claim 1 or 2, wherein a starting mixture is used in which the amount of isobutane is in the range of 50 to 95% by weight.

4. Process as claimed in any one of claims 1 to 3, wherein a starting mixture is used in which the amount of water is in the range of 0.3 to 3% by weight.

5. Process as claimed in any one of claims 1 to 4, wherein a starting mixture is used, additionally containing tertiary-butanol, preferably in an amount in the range of 5 to 15% by weight.

6. Process as claimed in any one of claims 1 to 5, wherein a starting mixture is used, originating from a process for the oxidation of isobutane.

7. Process as claimed in any one of claims 1 to 6, wherein the temperature in the distillation zone is in the range of 45 to 100 °C, and the pressure in the distillation zone is in the range of 1 to 10 bar gauge.

8. Process as claimed in claim 7, wherein the temperature in the distillation zone is in the range of 55 to 75 °C and the pressure in the distillation zone is in the range of 3 to 7 bar gauge.

9. Process as claimed in any one of claims 1 to 8, wherein the distillation zone is a distillation column, having from 10 to 25 theoretical trays.

10. Process as claimed in any of claims 6 to 9, characterized in that at least part of the overhead stream from the distillation zone is recycled to the process for the oxidation of isobutane.

## Patentansprüche

1. Verfahren zur Herstellung von tert.Butylhydroperoxid enthaltenden Gemischen, die zur Verwendung als Einsatzmaterial in der Epoxidation von Olefinen geeignet sind, worin ein Ausgangsgemisch, das wenigstens tert.Butylhydroperoxid, Isobutan und Wasser enthält, in eine Destillationszone eingeführt wird, wobei die Bedingungen in dieser Destillationszone derart ausgewählt werden, daß ein Überkopfstrom gebildet wird, der einen Teil des Isobutans und einen Hauptteil des Wassers enthält, und ein Bodenstrom gebildet wird, der den restlichen Teil des Isobutans und im wesentlichen das gesamte tert.Butylhydroperoxid enthält.

2. Verfahren nach Anspruch 1, worin ein Ausgangsgemisch verwendet wird, worin die Menge an tert.Butylhydroperoxid im Bereich von 5 bis 30 Gew.-% liegt.

3. Verfahren nach Anspruch 1 oder 2, worin ein Ausgangsgemisch verwendet wird, worin die Menge an Isobutan im Bereich von 50 bis 95 Gew.-% liegt.

4. Verfahren nach einem der Ansprüche 1 bis 3, worin ein Ausgangsgemisch verwendet wird, worin die Wassermenge im Bereich von 0,3 bis 3 Gew.-% liegt.

5. Verfahren nach einem der Ansprüche 1 bis 4, worin ein Ausgangsgemisch verwendet wird, das zusätzlich tert.Butanol, vorzugsweise in einer Menge im Bereich von 5 bis 15 Gew.-%, enthält.

6. Verfahren nach einem der Ansprüche 1 bis 5, worin ein Ausgangsgemisch verwendet wird, das aus einem Verfahren zur Oxidation von Isobutan stammt.

7. Verfahren nach einem der Ansprüche 1 bis 6, worin die Temperatur in der Destillationszone im Bereich von 45 bis 100°C liegt und der Druck in der Destillationszone im Bereich von 1 bis 10 bar Überdruck liegt.

8. Verfahren nach Anspruch 7, worin die Temperatur in der Destillationszone im Bereich von 55 bis 75 °C liegt und der Druck in der Destillationszone im Bereich von 3 bis 7 bar Überdruck liegt.

9. Verfahren nach einem der Ansprüche 1 bis 8, worin die Destillationszone eine Destillationskolonne mit 10 bis 25 theoretischen Böden ist.

10. Verfahren nach einem der Ansprüche 6 bis 9, dadurch gekennzeichnet, daß wenigstens ein Teil des Uberkopfstroms aus der Destillationszone zum Verfahren der Isobutanoxidation recycliert wird.

## Revendications

1. Procédé de préparation de mélanges contenant de l'hydroperoxyde de butyle tertiaire, qui conviennent à l'utilisation comme charge de départ pour l'époxydation d'oléfines, conformément auquel on introduit un mélange de départ contenant au moins de l'hydroperoxyde de butyle tertiaire, de l'isobutane et de l'eau, dans une zone de distillation, les conditions dans la zone de distillation étant choisies de manière à ce que se forme un courant de tête, contenant une partie de l'isobutane et la majeure partie de l'eau et un courant de fond ou de queue contenant la partie résiduelle de l'isobutane et sensiblement la totalité de l'hydroperoxyde de butyle tertiaire.

2. Procédé suivant la revendication 1, caractérisé en ce que l'on utilise un mélange de départ dans lequel la proportion d'hydroperoxyde de butyle tertiaire varie de 5 à 30% en poids.

3. Procédé suivant la revendication 1 ou 2, caractérisé en ce que l'on utilise un mélange de départ dans lequel la quantité d'isobutane varie de 50 à 95% en poids.

4. Procédé suivant l'une quelconque des revendications 1 à 3, caractérisé en ce que l'on utilise un mélange de départ dans lequel la proportion d'eau varie de 0,3 à 3% en poids.

5. Procédé suivant l'une quelconque des revendications 1 à 4, caractérisé en ce que l'on utilise un mélange de départ contenant en outre du butanol tertiaire, de préférence, en une proportion de 5 à 15% en poids.

6. Procédé suivant l'une quelconque des revendications 1 à 5, caractérisé en ce que l'on utilise un mélange de départ qui tire son origine de l'oxydation de l'isobutane.

7. Procédé suivant l'une quelconque des revendications 1 à 6, caractérisé en ce que la température dans la zone de distillation varie de 45 à 100°C et la pression dans la zone de distillation varie de 1 à 10 bars manométriques.

8. Procédé suivant la revendication 7, caractérisé en ce que la température dans la zone de distillation fluctue de 55 à 75°C et la pression dans la zone de distillation varie de 3 à 7 bars manométriques.

9. Procédé suivant l'une quelconque des revendications 1 à 8, caractérisé en ce que la zone de distillation est une colonne de distillation possédant de 10 à 25 plateaux théoriques.

10. Procédé suivant l'une quelconque des revendications 6 à 9, caractérisé en ce qu'au moins une partie du courant de tête de la zone de distillation est renvoyée au processus d'oxydation de l'isobutane.
